# FASCICULE DE BREVET EUROPEEN

(11) **EP 1 875 883 B1**
(45) Date de publication et mention de la délivrance du brevet: **18.04.2012**
(21) Numéro de dépôt: 07290788.4
(22) Date de dépôt: 26.06.2007
(51) Int. Cl.: A61F 2/86

(54) **Procédé de fabrication par moulage d'une endoprothèse en matériau polymère**
Verfahren zur Eingussherstellung einer Endoprothese aus Polymermaterial
Moulding manufacturing method of an endoprosthesis using a polymer material

(30) Priorité: 04.07.2006 FR 0606080
(43) Date de publication de la demande: 09.01.2008
(73) Titulaire: MS Techniques, 54340 Pompey (FR)
(72) Inventeur: Malher, Etienne, 54000 Nancy (FR); Siegel, Sylvain, 54000 Nancy (FR)
(74) Mandataire: Lagrange, Jacques Etienne M.M.

(56) Documents cités:
- WO-A-2004/069097
- WO-A-2005/042057
- US-A- 6 010 530
- US-A1- 2004 158 275
- US-B1- 6 203 732

## Description

La présente invention est relative à la fabrication d'une endoprothèse en matériau polymère.

Les endoprothèses sont des implants médicaux de forme généralement tubulaire, destinées à maintenir ouvert un canal qui à tendance à se refermer sous l'effet d'une pathologie, telles qu'une tumeur, une plaque d'athérome, etc... Le canal qui doit être maintenu ouvert est par exemple une artère, une veine, une voie respiratoire, une voie urinaire ou un canal biliaire.

Afin de pouvoir être mise en place facilement, les endoprothèses doivent être déformables entre un état contracté et un état dilaté. En effet, les endoprothèses sont introduites dans les canaux dans un état contracté afin d'être amené jusqu'à leur lieu d'implantation de façon peu traumatiques, et elles sont ensuite dilatées à leur dimension définitive soit par déformation plastique à l'aide d'un outillage adapté, soit par un retour élastique.

On connaît des endoprothèses en acier inoxydable ou en alliage nickel titane dont la forme générale est celle d'une tresse tubulaire ou d'un tube fortement ajouré ne laissant qu'un mince réseau de bras métalliques agencés de manière à obtenir une bonne souplesse en position contractée et une bonne tenue mécanique après déploiement. Elles peuvent être fabriquées par tressage d'un fil métallique, par découpage d'un tube métallique ayant le diamètre final de l'implant en mode expansé, ou par découpage d'un tube métallique ayant le diamètre de l'implant en mode contracté. En outre, elles peuvent avoir subi un traitement de surface destinée à fixer à la surface des molécules ayant un effet thérapeutique.

On connaît aussi le procédé de moulage d'une endoprothèse décrit dans le document US 2004158275 en ce qu'on moule l'endoprothèse sur un rouleau matrice dont la surface externe comporte un empreinte en creux ayant la forme de l'endoprothèse. Le rouleau matrice étant entraîné en rotation autour de son axe; on laisse l'endoprothèse se solidifier, puis on la retire du rouleau matrice.

Ces endoprothèses métalliques présentent l'inconvénient d'une part d'être coûteuses à réaliser, d'autre part de ne permettre que des traitements de surface limités et enfin d'être inaltérables, ce qui a pour conséquence qu'il n'est possible des les enlever qu'en les retirant par une opération adaptée.

Afin de remédier à ces inconvénients, on a proposé de réaliser des endoprothèses en matériau polymère qui ont l'avantage d'avoir une meilleure biocompatibilité que les matériaux métalliques utilisés habituellement, de permettre de réaliser des greffages chimiques de molécules ayant des effets thérapeutiques tels que des inhibiteurs de re-sténose ou des anti-coagulants et d'être plus économiques à réaliser. En outre, à condition d'utiliser un polymère résorbable, il est possible de fabriquer des endoprothèses qui disparaissent au-delà d'une certaine durée, ce qui élimine le risque de resténose provoquée par un corps étranger.

La fabrication des endoprothèses polymères présente cependant des difficultés. En effet, du fait de la forme de ces endoprothèses, et en particulier des bras qui les constituent et qui ont des sections très faibles tout en ayant des longueurs relativement importantes, il est difficile d'utiliser des techniques de moulage classique pour obtenir un bon remplissage des moules et des endoprothèses ayant des formes satisfaisantes. En particulier, du fait de la forme complexe des endoprothèses, un moulage par injection classique engendrerait la présence de zones de re-soudure très nombreuses qui constitueraient des points de fragilité. Par ailleurs, il peut être souhaitable, pour des questions de résistance au vieillissement, d'utiliser des polymères de poids moléculaire élevé, qui ont des viscosités à l'état fondu importantes, et qui rendent le moulage d'autant plus difficile.

Le but de la présente invention est de remédier à ces inconvénients en proposant un moyen pour fabriquer par moulage des endoprothèses en matériau polymère, d'une façon plus économique, plus sûre et permettant d'utiliser des polymères plus visqueux que les procédés de moulage connus.

A cet effet, l'invention a pour objet un procédé de fabrication par moulage d'une endoprothèse en matériau polymère, selon lequel on moule l'endoprothèse sur un rouleau matrice dont la surface externe comporte une empreinte en creux ayant la forme de la prothèse, le rouleau matrice étant entraîné en rotation autour de son axe, on laisse l'endoprothèse se solidifier, puis on retire l'endoprothèse du rouleau matrice.

On serre contre le rouleau matrice un contre rouleau d'axe parallèle à l'axe du rouleau matrice, entraîné en rotation en sens inverse du rouleau matrice, afin d'assurer le bon remplissage de l'empreinte, et on enlève l'excédent de matériau polymère par exemple en raclant la surface du rouleau matrice à l'aide d'un racloir ayant une arête parallèle à l'axe du rouleau matrice

De préférence, pendant le remplissage du rouleau matrice, on chauffe au moins le rouleau matrice à une température comprise entre la température du fusion et la température de dégradation du polymère.

De préférence, après remplissage du rouleau matrice, on refroidit au moins le rouleau matrice pour laisser l'endoprothèse se solidifier.

On peut apporter le matériau sous forme de granulé ou de poudre qu'on fait fondre au contact du rouleau matrice.

On peut, également, apporter le matériau polymère à l'état fondu.

Après remplissage du rouleau matrice, on peut dégager le rouleau matrice pour laisser solidifier l'endoprothèse et la retirer, et remettre en place un autre rouleau matrice qu'on remplit de matériau polymère.

On peut effectuer le moulage dans une enceinte contenant une atmosphère inerte.

L'invention concerne également un dispositif pour la fabrication par moulage d'une endoprothèse en matériau polymère par le procédé selon l'invention. Ce dispositif comprend un rouleau matrice dont la surface comporte une empreinte ayant la forme de l'endoprothèse et un contre rouleau, parallèles entre eux, montés rotatifs autour de leur axes respectifs et montés mobiles l'un par rapport à l'autre de façon à pouvoir être écartés ou rapprochés, des moyens d'entraînement en rotation et des moyens pour déplacer les rouleaux, des moyens de chauffage et de refroidissement des rouleaux, un moyen de raclage de la surface du rouleau matrice et un moyen d'alimentation en matériau polymère.

Le rouleau matrice, le contre rouleau et le moyen de raclage peuvent être disposés dans une enceinte contenant une atmosphère inerte.

L'invention va maintenant être décrite de façon plus précise mais non limitative en regard des figures annexées dans lesquelles :
- la figure 1 est une vue en perspective d'une endoprothèse en matériau polymère ;
- la figure 2 est une vue schématique d'une installation de moulage d'une endoprothèse en matériau polymère ;

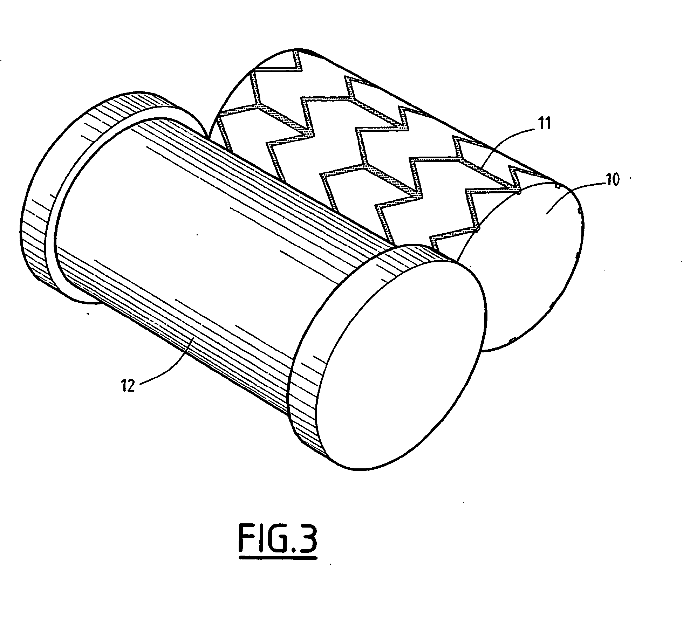

- la figure 3 est une vue en perspective d'un dispositif de moulage d'une endoprothèse comportant un moule cylindrique.

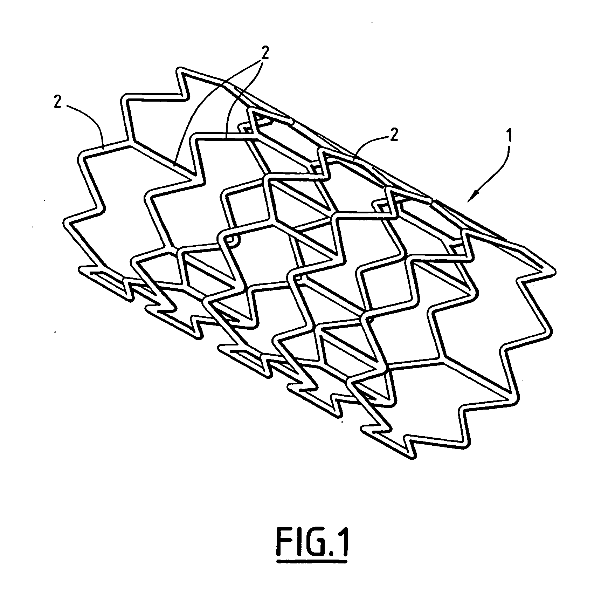

L'endoprothèse, repérée généralement par 1 à la figure 1, est un tube fortement ajouré formé de bras 2, de petit diamètre et de longueur relativement importante par rapport au diamètre, disposés en réseau de façon à former une paroi ajourée cylindrique.

Les bras sont disposés de façon à former des bras ayant des angles afin que la surface cylindrique soit élastique et puisse être déformée entre une position contractée et une position dilatée. Une telle endoprothèse, connue en elle-même, est réalisée en un matériau polymère thermo plastique biocompatible, connu en lui-même.

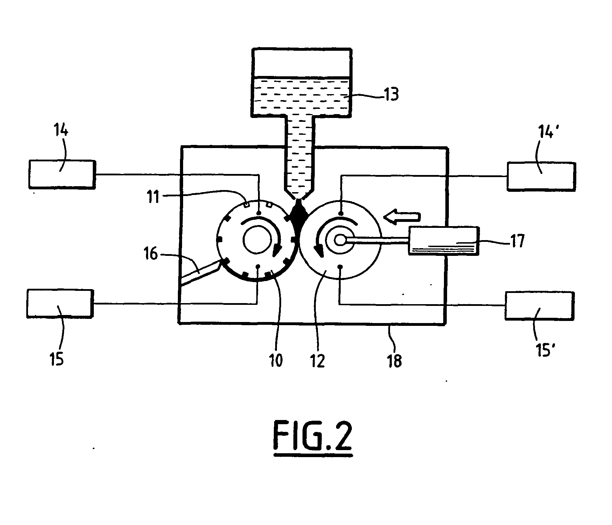

Pour fabriquer l'endoprothèse, représentée à la figure 1, on utilise un procédé de moulage à l'aide d'un dispositif de moulage, représenté à la figure 2, qui comporte un rouleau matrice 10 de forme cylindrique dont la surface externe comporte une empreinte en creux 11 ayant la forme d'une endoprothèse dans une position par exemple expansée, et un contre rouleau 12 d'axe parallèle au rouleau matrice 10, disposé à proximité du rouleau matrice.

Le rouleau matrice 10 et le contre rouleau 12 ont des axes horizontaux, parallèles entre eux, de façon à pouvoir coopérer par l'intermédiaire d'une génératrice.

Le dispositif de moulage comporte également un moyen 13 d'alimentation en matériau polymère disposé de façon à pouvoir apporter du matériau polymère dans la zone de contact du rouleau matrice et du contre rouleau.

Le moyen d'approvisionnement en matériau polymère 13 peut être soit un distributeur de matériau polymère granulé ou en poudre, soit un dispositif d'approvisionnement en matériau polymère déjà fondu telle qu'un extrudeuse.

Le rouleau matrice 10 comporte un moyen 14 de chauffage et un moyen 15 de refroidissement.

Le moyen 14 de chauffage est par exemple un dispositif à circulation de fluides chauds, telle que l'eau chaude, ou encore un moyen de chauffage par résistance électrique.

Le moyen de refroidissement 15 est par exemple un dispositif de circulation d'eau froide.

Les moyens de chauffage 14 et de refroidissement 15 peuvent être pilotés de façon à pouvoir successivement chauffer le rouleau 10 puis le refroidir.

Le contre rouleau 12 comporte également un moyen de chauffage 14' et un moyen de refroidissement 15' qui permettent de le chauffer et de le refroidir. Ces moyens sont semblables à ceux du rouleau matrice.

En outre, le contre rouleau 12 est monté sur un support mobile entraîné par un moyen de déplacement 17, tel qu'un vérin, permettant de le déplacer horizontalement et de régler sa distance par rapport au rouleau matrice. Le moyen de déplacement 17 permet également de régler et éventuellement la pression de contact avec le rouleau matrice.

Le rouleau matrice 10 et le contre rouleau 12 comportent des moyens d'entraînement en rotation, non représentés, tels que des moteurs électriques, et qui permettent de faire tourner les deux rouleaux en sens inverse l'un de l'autre à des vitesses qui peuvent être différentes.

Le dispositif de moulage comporte en outre un moyen de raclage 16 constitué d'une lame pouvant être amenée en contact de la surface du rouleau matrice.

Enfin, l'ensemble est contenu dans une enceinte 18 qui peut être remplie d'un gaz neutre tel que de l'azote.

On va maintenant décrire la fabrication d'une endoprothèse avec une installation alimentée en granulée ou en poudre solide de polymère.

Tout d'abord, on porte le rouleau moule et le contre rouleau à une température supérieure à la température de fusion du polymère et inférieure à sa température de dégradation. Puis, on rapproche les rouleaux l'un de l'autre sans toutefois qu'ils se touchent.

On met en rotation les deux cylindres à des vitesses opposées, de telle sorte que les vitesses linéaires à la surface des cylindres soient sensiblement égales, ou légèrement différentes selon que l'on veut éviter ou obtenir un effet de cisaillement.

A l'aide du moyen d'approvisionnement 13 on apporte des granulés ou des poudres de polymère au-dessus de la zone de contact des deux cylindres en quantité légèrement supérieure à la quantité nécessaire pour remplir l'empreinte du rouleau matrice. Sous l'effet de la température du rouleau matrice et du contre rouleau, le polymère sous forme de granulés ou de poudre fond, se répand et vient recouvrir la surface du rouleau matrice. Lorsque le polymère fondu est bien réparti sur l'ensemble de la longueur du rouleau matrice, les rouleaux sont rapprochés l'un de l'autre pour assurer un remplissage parfait de l'empreinte du rouleau matrice. On approche alors le racloir 16 de façon à ce qu'il vienne racler la surface du rouleau matrice et retirer l'excédent de matériau polymère à l'état fondu.

On refroidit alors le rouleau matrice et éventuellement le contre rouleau de façon à permettre la solidification du matériau polymère qui remplit l'empreinte du rouleau matrice.

Lorsque le matériau, qui remplit l'empreinte du rouleau matrice et qui constitue donc une endoprothèse, est solidifié, on écarte le rouleau matrice et on retire l'endoprothèse du rouleau matrice.

Pendant toutes ces opérations, l'enceinte peut être remplie d'un gaz neutre tel que de l'azote, afin d'éviter des réactions chimiques entre le polymère et l'atmosphère dans lequel se déroule le moulage. Cette atmosphère inerte est particulièrement utile lorsque le polymère est sensible à l'oxydation ou à l'hydrolyse.

L'installation qui vient d'être décrite peut comporter une pluralité de rouleaux matrice montés sur un dispositif permettant de changer le rouleau matrice, ce qui permet de retirer le rouleau matrice en fin de remplissage de façon à effectuer la solidification en dehors de la zone de remplissage, et pendant la solidification d'un rouleau matrice dont l'empreinte est remplie, de le remplacer dans la zone de remplissage par un nouveau rouleau matrice. Cette disposition à l'avantage d'augmenter la productivité en effectuant la solidification en temps masqué.

Dans une variante de ce procédé de fabrication d'endoprothèse, on utilise un dispositif d'alimentation 13 chauffant permettant d'apporter du polymère déjà fondu dans la zone de contact du rouleau matrice et du contre rouleau. Le polymère étant déjà fondu, le contact du matériau polymère avec les rouleaux chauffés peut être considérablement réduit puisqu'il n'y a plus besoin de faire fondre le matériau polymère. Ainsi il est possible d'augmenter sensiblement la productivité.

Ce procédé à l'avantage d'avoir un très bon rendement et d'en outre utiliser des quantités de matière faible, ce qui est un avantage notamment lorsque les prothèses sont réalisées en polymères résorbables qui sont des polymères très coûteux.

Ce procédé à l'avantage en outre de réduire le temps de séjour à chaud du matériau, et donc de réduire la dégradation qui peut résulter de son séjour à haute température.

Bien évidemment, les conditions de chauffage des rouleaux et de vitesse relatives doivent être adaptées en fonction de la nature des polymères et de la forme précise de l'endoprothèse que l'on doit réalisée, et l'homme du métier sait faire les adaptations nécessaires.

## Revendications

1. Procédé de fabrication par moulage d'une endoprothèse (1) en matériau polymère, selon lequel on moule l'endoprothèse sur un rouleau matrice (10) dont la surface externe comporte une empreinte (11) en creux ayant la forme de la prothèse, le rouleau matrice étant entraîné en rotation autour de son axe, on laisse l'endoprothèse se solidifier, puis on retire l'endoprothèse du rouleau matrice, **caractérisé en ce qu'**on serre contre le rouleau matrice (10), un contre rouleau (12) d'axe parallèle à l'axe du rouleau matrice, entraîné en rotation en sens inverse du rouleau matrice, afin d'assurer le bon remplissage de l'empreinte, et **en ce qu'**on enlève l'excédent de matériau polymère en raclant la surface du rouleau matrice

2. Procédé selon la revendication 1, **caractérisé en ce que**, pendant le remplissage du rouleau matrice (10), on chauffe au moins le rouleau matrice (10) à une température comprise entre la température de fusion et la température de dégradation du polymère.

3. Procédé selon la revendication 2, **caractérisé en ce que**, après remplissage du rouleau matrice (10), on refroidit au moins le rouleau matrice (1) pour laisser l'endoprothèse se solidifier.

4. Procédé selon l'une quelconque des revendications 1 à 3, **caractérisé en ce qu'**on apporte le matériau sous forme de granulé ou de poudre qu'on fait fondre au contact du rouleau matrice (10).

5. Procédé selon l'une quelconque des revendication 1 à 3, **caractérisé en ce qu'**on apporte le matériau polymère à l'état fondu.

6. Procédé selon l'une quelconque des revendications précédentes, **caractérisé en ce que**, après remplissage du rouleau matrice (10), on dégage le rouleau matrice pour laisser solidifier l'endoprothèse et la retirer, et on remet en place un autre rouleau matrice qu'on remplit de matériau polymère.

7. Procédé selon l'une quelconque des revendication 1 à 6, **caractérisé en ce qu'**on effectue le moulage dans une enceinte (18) contenant une atmosphère inerte.

8. Dispositif pour la fabrication par moulage d'une endoprothèse (1) en matériau polymère par le procédé selon l'une quelconque des revendications 1 à 7, **caractérisé en ce qu'**il comprend un rouleau matrice (10) dont la surface comporte une empreinte (11) ayant la forme de l'endoprothèse et un contre rouleau parallèles entre eux, montés rotatifs autour de leur axes respectifs et montés mobiles l'un par rapport à l'autre de façon à pouvoir être écartés ou rapprochés, des moyens d'entraînement en rotation et des moyens (17) pour déplacer les rouleaux, des moyens de chauffage (14, 14') et de refroidissement (15, 15') des rouleaux, un moyen de raclage (16) de la surface du rouleau matrice et un moyen (13) d'alimentation en matériau polymère.

9. Dispositif selon la revendication 8, **caractérisé en ce que** le rouleau matrice (10), le contre rouleau (12) et le moyen de raclage sont disposés dans une enceinte (18) contenant une atmosphère inerte.

## Claims

1. A method of fabricating a polymer material endoprosthesis (1) by molding, the method comprising: molding the endoprosthesis on a matrix roller (10) having an outer surface that includes a recess (11) in the shape of the prosthesis, the matrix roller being rotated about its axis; allowing the endoprosthesis to solidify; and then withdrawing the endoprosthesis from the matrix roller, **characterized in that** a backing roller (12) is pressed against the matrix roller (10), the backing roller being of axis parallel to the axis of the matrix roller, the backing roller being rotated in she opposite direction to the matrix roller and serving to ensure that the recess is properly filled and **in that** the excess polymer material is removed by scraping the surface of the matrix roller.

2. A method according to claim 1, **characterized in that** while the matrix roller (10) is being filled, at least the matrix roller (10) is heated to a temperature lying between the melting temperature and the degradation temperature of the polymer.

3. A method according to claim 2, **characterized in that** after the matrix roller (10) has been filled, at least the matrix roller is cooled in order to allow the endoprosthesis (1) to solidify.

4. A method according to any of claims 1 to 3, **characterized in that** the material is fed in the form of granules or powder and is caused to melt in contact with the matrix roller (10).

5. A method according to any of claims 1 to 3, **characterized in that** the polymer material is fed in the molten state.

6. A method according to any of the preceding claims , **characterized in that** after the matrix roller (10) has been filled, the matrix roller is moved away to allow the endoprosthesis to solidify and be withdrawn, and another matrix roller takes its place and is filled with polymer material.

7. A method according to any of claims 1 to 6, **characterized in that** the molding is performed in an enclosure (18) containing an inert atmosphere.

8. A device for fabricating a polymer material endoprosthesis (1) by molding by using the method according to any of claims 1 to 7, **characterized in that** it comprises a matrix roller (10) having a surface including a recess (11) in the form of the endoprosthesis, and a backing roller that is parallel-thereto, the rollers being mounted to rotate about their respective axes and being movable relative to each other so as to be capable of being moved apart or towards each other, means for driving the rollers in rotation, and means (17) for moving the rollers , heater and cooler means (15, 15') for the rollers, scraper means (16) for scraping the surface of the matrix roller, and polymer material feeder means (13).

9. A device according to claim 8, **characterized, in that** the matrix roller (10), the backing roller (12), and the scraper means are disposed in an enclosure (18) containing an inert atmosphere.

## Patentansprüche

1. Verfahren zum Herstellen durch Gießen eine Endoprothese (1) aus Polymermaterial, wobei die Endoprothese auf eine Gesenkwalze (10) gegossen wird, deren äußere Oberfläche eine Hohlprägung aufweist, die die Form der Prothese hat, wobei die Gesenkwalze um ihre Achse rotatorisch angetrieben wird, wobei zugelassen wird, dass sich die Endoprothese verfestigt, und wobei dann die Endoprothese von der Gesenkwalze abgenommen wird, **dadurch gekennzeichnet, dass** gegen die Gesenkwalze (10) eine Gegenwalze (12) mit zu der Achse der Gesenkwalze paralleler Achse gedrängt wird und in dem zu der Gesenkwalze entgegengesetzten Richtungssinn rotatorisch angetrieben wird, um ein gutes Befüllen der Prägung sicherzustellen, und dass überschüssiges Polymermaterial abgetragen wird, indem die Oberfläche der Gesenkwalze abgeschabt wird.

2. Verfahren nach Anspruch 1, **dadurch gekennzeichnet, dass** während des Befüllens der Gesenkwalze (10) wenigstens die Gesenkwaize (10) auf eine Temperatur erwärmt wird, die im Bereich von der Schmelzlemperatur bis zu der Verschlechterangstemperatur des Polymers liegt.

3. Verfahren nach Anspruch 2, **dadurch gekennzeichnet, dass** nach dem Bcfüllen der Gesenkwalze (10) wenigstens die Gesenkwatze (1) abgekühlt wird, damit sich die Endoprothese verfestigen kann.

4. Verfahren nach einem der Ansprüche 1 bis 3, **dadurch gekennzeichnet, dass** das Material in Korn- oder Pulverform eingebracht wird, das in Kontakt mit der Gesenkwalze (10) geschmolzen wird.

5. Verfahren nach einem der Anspruche 1 bis 3, **dadurch gekennzeichnet, dass** das Polymermaterial im geschmolzenen Zustand eingebracht wird.

6. Verfahren nach einem der vorhergehenden Ansprüche, **dadurch gekennzeichnet, dass** nach dem Befüllen der Gesenkwalze (10) die Gesenkwalze gelöst wird, damit sich die Endoprothese verfestigen kann und um sie abnehmen zu können, und dass eine andere Gesenk walze wieder angeordnet wird, die mit Polymermaterial gefüllt wird.

7. Verfahren nach einem der Ansprüche 1 bis 6, **dadurch gekennzeichnet, dass** das Gießen in einem Behälter (18), der eine Inertatmosphäre enthält, erfolgt.

8. Vorrichtung für die Herstellung durch Gießen einer Endoprothese (1) aus Polymermaterial durch das Verfahren nach einem der Ansprüche 1 bis 7, **dadurch gekennzeichnet, dass** sie umfasst: eine Gesenk walze (10), deren Oberfläche eine Prägung (11) aufweist, die die Form der Endoprothese hat, und eine Gegenwalze, die zu der Gesenkwalze (10) parallel ist, die um ihre jeweiligen Achsen drehbar montiert sind und beweglich in Bezug aufeinander montiert sind, derart, dass sie voneinander beabstandet und aneinander angenähert werden können, und Mittel zum rotatorischen Antrieb, Mittel (17) zum Verlagern der Walzen, Mittel zum Erwärmen (14, 14') und zum Kühlen (15, 15') der Walzen, ein Mittel zum Abschaben (16) der Oberfläche der Gesenkwalze und ein Mittel (13) zum Versorgen mit Polymermaterial.

9. Vorrichtung nach Anspruch 8, **dadurch gekennzeichnet, dass** die Gesenkwalze (30), die Gegenwalze (12) und das Abschabmittel in einem Behälter (18) angeordnet sind, der eine Inertatmosphäre enthält.
